# EUROPEAN PATENT APPLICATION

(11) **EP 1 905 838 A1**
(43) Date of publication of application: **02.04.2008**
(21) Application number: 06020561.4
(22) Date of filing: 29.09.2006
(51) Int. Cl.: C12P 19/12

(54) **Enzymatic regioselective 6-acylation of sucrose**

(71) Applicant: DSM IP Assets B.V., 6411 TE Heerlen (NL)
(72) Inventor: Quaedflieg, Peter Jan Leonard Mario, 6181 KA Elsloo (NL); Kierkels, Joannes Gerardus Theodorus, 6133 BH Sittard (NL); Welten-Schoevaars, Anne Marie, 49160 St. Martin de la Place (FR)
(74) Representative: Verhaegen, Ilse Maria M.

(57) **Abstract**

The invention relates to a process for the enzymatic preparation of 6-O-acylsucrose (Figure 1) or a derivative thereof 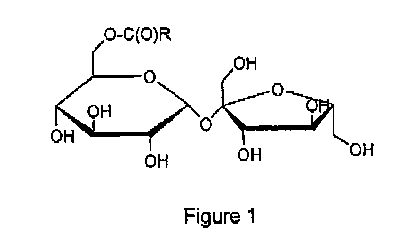 in the presence of at least one non-nucleophilic organic solvent, by selective monoacylation of sucrose on the 6-position with a donor acyl ester of formula R¹R²C=CR³-O-C(O)R, in which R¹, R², and R³ each independently represent hydrogen, an optionally substituted (hetero)alkyl group or an optionally substituted (hatero)aryl group with for example 1-50 C atoms, or of formula R⁴-O-C(O)R, in which R⁴ is a halogenated alkyl group of 2-50 carbon atoms in which at least the 2-position of the alkyl group is substituted with one, two or three halogens, and with R representing an optionally substituted (hetero)alkyl or (hetero)aryl group having from 1 to 50 carbon atoms, wherein the acylation is carried out in the presence of a lipase enzyme selected from the group of
(i) lipases obtainable from a micro-organism from a *Surkholderia gladioli* species; or
(ii) any such *Burkholderia gladioli* lipase enzyme produced and over-expressed in another suitable micro-organism; or
(iii) mutants of the lipases from (i) and/or (ii) having selective acylation activity to form 6-O-acylsucrose in acylation of sucrose.

## Description

The invention relates to a process for the enzymatic preparation of 6-*O*-acylsucrose (Figure 1 with R representing an optionally substituted (hetero)alkyl or (hetero)aryl group having from 1 to 50 carbon atoms) or a derivative thereof 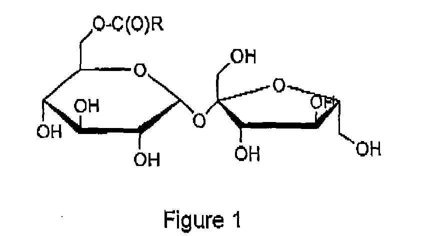 by selective monoacylation of sucrose at the 6-position with a donor acyl ester of formula R¹R²C=CR³-O-C(O)R, in which R¹, R², and R³ each independently represent hydrogen, an optionally substituted (hetero)alkyl group or an optionally substituted (hetero)aryl group with for example 1-50 C atoms and in which R has the meaning defined above, or of formula R⁴-O-C(O)R, in which R⁴ is a halogenated alkyl group of 2-50 carbon atoms in which at least the 2-position of the alkyl group is substituted with one, two or three halogens, in the presence of an enzyme and at least one non-nucleophilic organic solvent.

6-*O*-acylsucrose has a considerable interest as an intermediate product in the synthesis of the synthetic intense sweetener sucralose (4,1',6'-trichloro-4,1',6'-trideoxygalactosucrose, Figure 2).

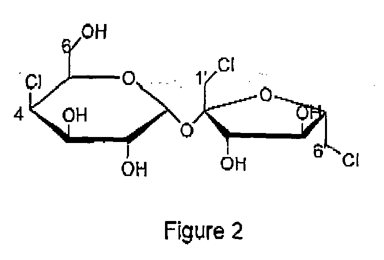

Sucralose is approximately 2.5 times sweeter than aspartame (α-L-aspartyl-L-phenylalanine methyl ester) and has the advantage that it is suitable for a number of products for which aspartame is less suitable, e.g. food products that need to undergo heat treatment, have an unfavourable pH for aspartame and/or need to have a very long shelf life.

In general, enzymatically catalysed acylations of sucrose in organic media usually result in low conversions to the desired product 6-*O*-acylsucrose, as is shown in, for example, M. Woudenberg-Van Oosterom et al., "Regioselective acylation of disaccharides in tert-butyl alcohol catalyzed by Candida antarctica lipase" Biotechnol. Bioeng. 49 (1996), 328-333. Herein enzymatic acylation is described of several disaccharides with ethyl butanoate or ethyl dodecanoate as the donor acyl ester, the acylation being catalyzed by *Candida antarctica* lipase. At relatively low conversion several monoesters resulting from acylation of the primary alcohol groups were obtained as the primary products, while at higher conversion diesters were formed, the ratio of diester to monoester being dependent on the structure of the disaccharide.

A process for the enzymatic preparation of 6-*O*-acetylsucrose is also known from US Patent 5,128,248, wherein sucrose is acetylated with isopropenylacetate in pyridine in the presence of lipase P (Amano).

A drawback of the process disclosed in US Patent 5,128,248 is the low conversion to the desired product 6-O-acetylsucrose, in spite of the large excess of acyl donor and large amounts of enzyme, as has been confirmed in Comparative Example B of the present application. Additionally, a significant amount of 4',6-di-O-acetylsucrose is formed as a by-product.

The object of the present invention is therefore to provide a commercially attractive process in which sucrose or a derivative thereof is acylated to 6-*O*-acylsucrose or a derivative thereof in a much higher conversion than known from the state of the art, the acylation reaction being catalysed by an enzyme.

Surprisingly, this object is achieved according to the invention by performing the process in the presence of a lipase enzyme selected from the group of
(i) lipases obtainable from a micro-organism from a *Burkholderia gladioli* species, or
(ii) any such *Burkholderia gladioli* lipase enzyme produced and overexpressed in another suitable micro-organism, or
(iii) mutants of the lipases from (i) and/or (ii) having selective acylation activity to form 6-*O*-acylsucrose in acylation of sucrose.

Accordingly, not only lipases naturally produced by a micro-organism from a *Burkholderia gladioli* species, whether or not they are obtained from a *Burkholderia gladioli* species micro-organism (or are obtained by heterologous over-expression in another suitable microorganism) can be used in the context of the present invention, but also such lipases can be used as are obtained by mutation of genes coding for such lipases to obtain mutant lipases having selective acylation activity to form 6-*O*-acylsucrose in acylation of sucrose. Of course, also any other lipases having similar activity with respect to the selective acylation of the 6-position of sucrose can be used in the process according to the invention.

Preferably a lipase produced by a micro-organism from a *Burkholderia gladioli* species is used, more preferably a lipase chosen from the group consisting of Lipase PL, Lipase QL, and Lipase QLM; most preferably, the lipase is Lipase QL or Lipase QLM. In the process according to the invention lipases can be used as such, as part of a whole cell (which may be a *Burkholderia gladioli* cell, or a cell of any other micro-organism in which the lipases from *Burkholderia gladioli* may be over-expressed), or immobilized on a solid carrier.

The lipase used in the process according to the invention is generally present in a quantity corresponding to 1.000-1.000.000 U per gram of sucrose or a derivative thereof. Preferably a quantity corresponding to between 10.000 and 100.000 U per gram of sucrose is used, more preferably between 25.000 and 75.000 U per gram of sucrose.

As used herein, the unit 1 U corresponds to the amount of enzyme which liberates 1 µmol of fatty acid per min at 37 °C in the assay method of Kokusho et al. (Agric. Biol. Chem. 46 (1982), 1159-1164) using olive oil emulsified with polyvinyl alcohol as a substrate (see: Naka Y., Yukagaku 36 (1987), 821-825).

The skilled person can easily establish the actual quantity in weight of the lipase to be used most suitably in the process of the invention. It will be clear that such quantity will depend on the activity of the lipase used (in U/gram lipase), which is determined by parameters like the purity and formulation (e.g. free or immobilized enzyme) of the lipase as used. Depending on the quantity of catalyst not only the reaction rate, but also the selectivity achieved, may vary. The skilled person will take these factors into account, when determining the optimum quantity in weight of the lipase to be used most suitably. One also may influence the reaction rate and its selectivity by providing suitable mutants of the lipases to be used in the context of the present invention.

Excellent results also may be achieved, if the lipase used is a lyophilised lipase, optionally in the presence of a lyoprotectant, i.e. of a component that protects the lipase during lyophilization. As the lyoprotectant a wide range of compounds can be used. Conveniently, the substrate sucrose itself, or derivatives thereof, as exemplified below, may be applied as lyoprotectant.

The substrate used in the process of the invention is either sucrose itself, or a derivative thereof, for example derivatives bearing one or more *O*-functional groups such as acyl or aroyl groups (such derivatives being esters); or alkyl or aryl groups (which types of derivatives are ethers); or silyl groups (i.e. these are silyl ethers); or derivatives wherein one or more of the hydroxyl groups other than the 6-hydroxy group are replaced by a halogen atom); in any case, however, in any of the derivatives at least the 6-position carries a free hydroxyl group.

In particular the method according to the invention can be used for the production of 6-*O*-acylsucrose, which is an intermediate in for example the preparation of sucralose from sucrose.

The starting concentration of substrate (i.e. of sucrose or a derivative thereof) in the reaction mixture is generally chosen between 0.1 and 60 wt.%, in particular between 1 and 40 wt.%, more in particular between 2 and 10 wt.%. The most suitable concentration of substrate, preferably of sucrose, depends on e.g. the solubility of sucrose (or the derivative thereof) in the reaction medium. It should be noted, however, that the concentration of sucrose or the derivative thereof is not limited to the maximum solubility of sucrose or the derivative thereof in the solvent at the temperature applied; the sucrose or the derivative thereof may not dissolve completely at the beginning of the reaction, but will dissolve completely in the course of the reaction when the dissolved sucrose or derivative thereof is transformed into the product 6-O-acylsucrose, because this compound generally has a higher solubility than the substrate.

A donor acyl ester of formula R¹R²C=CR³-O-C(O)R, in which R¹, R², R³, and R have the meaning as defined above, or a donor acyl ester of formula R⁴-O-C(O)R in which R⁴ has the meaning as defined above is used in the process according to the invention. Examples of heteroatoms in the optionally substituted (hetero)alkyl group and optionally substituted (hetero)aryl group with for example 1-50 C atoms which may be present in R, R¹, R² and R³ are N, O, P and S. Examples of substituents on the (hetero) alkyl group or (hetero) aryl group are a nitro group, a halogen, an alkyl group with 1-6 C atoms, and an alkoxy group with 1-6 C atoms.

Examples of donor acyl esters of formula R¹R²C=CR³-O-C(O)R are isopropenyl and vinyl esters, for example vinyl acetate, vinyl proprionate, vinyl butyrate, and isopropenyl acetate. In particular when R = CH₃, an isopropenyl ester is preferred rather than a vinyl ester, the isopropenyl group usually having a positive effect on the selectivity of the acylation reaction, as isopropenyl esters are usually hardly reactive in the presence of sucrose or a derivative thereof in the absence of an enzyme. In other cases, when the bulky isopropenyl group of isopropenyl esters is not required to suppress undesired side-reactions in the absence of an enzyme, vinyl esters are preferred, thereby usually leading to higher reaction rates.

Examples of donor acyl esters of formula R⁴-O-C(O)R are are 2,2,2-trichloroethylesters and 2,2,2-trifluoroethylesters.

The enzymatic acylation of sucrose to 6-*O*-acylsucrose by using hydrophobic vinyl esters of fatty acids as the acylating agent is commonly known to result in reasonable conversions, for example from M. Ferrer et al., Biotechnol. Bioeng., 65 (1999), 10, and W. Tzuzuki et al., Biotechol. Bioeng., 64 (1999), 267. On the other hand, it is also commonly known that the use of short chain, relatively hydrophilic donor acyl esters usually results is much lower conversions to the desired product. See for example J.O. Rich et al., Biotechnology and Bioengineering, 45 (1995), 426-434. It is therefore even more surprising that high conversions to 6-*O-*acylsucrose or derivatives thereof are obtained even when the reaction is performed using a short chain donor acyl ester, for example vinyl acetate and isopropenyl acetate.

The molar ratio between the donor acyl ester and sucrose or a derivative thereof is not particularly critical and will generally be chosen between 0.5:1 and 100:1, preferably between 0.8:1 and 50:1, more preferably between 1:1 and 25:1, in particular between 2:1 and 10:1. For economic reasons the molar ratio between the donor acyl ester and sucrose or a derivative thereof is in particular between 2:1 and 6:1. The donor acyl ester may also be used as a solvent. In that case the ratio between the donor acyl ester and sucrose or a derivative thereof may be higher than 100:1.

The process according to the invention is carried out in the presence of at least one non-nucleophilic organic solvent which has sufficient polarity to dissolve at least part of each reactant, i.e. sucrose or a derivative thereof and the donor acyl ester, under the applied reaction conditions, for example 3 wt.% at 30 °C. Non-nucleophilic-solvents is defined here to describe solvents that do not compete with the substrate by reacting with the donor acyl esters. Preferably the solvent used is selected from the group of non-nucleophilic amines, non-nucleophilic amides, non-nucleophilic sterically hindered alcohols, and non-nucleophilic sulfoxides. Examples of non-nucleophilic amines are tertiary amines, such as *N*-methyl morpholine or triethyl amine, or aromatic amines such as pyridine. In particular aromatic amines are preferred, for example pyridine. Examples of non-nucleophilic amides are *N*,*N*-dimethyl formamide and 1-methyl-2-pyrrolidone (NMP). Examples of sterically hindered alcohols, which owe their non-nucleophilicity to their bulky nature, are t-butanol and t-amylalcohol (2-mathyl-2-butanol). Examples of non-nucleophilic sulfoxides are, for instance, dimethyl sulfoxide and sulfolane.

The process according to the invention may also be carried out in a mixture of solvents, for example a mixture of two or more of the non-nucleophilic solvents mentioned above. In addition to the non-nucleophilic solvents, however, also one or more hydrophobic solvents (i.e. co-solvents) may be present, thereby forming a mixture of one or more of the non-nucleophilic solvents mentioned above and one or more hydrophobic co-solvents. Examples of suitable hydrophobic solvents include for example optionally substituted aliphatic or aromatic hydrocarbons with for example 5-12 carbon atoms. The presence of one or more of such hydrophobic solvents may result in a higher conversion to 6-*O*-acylsucrose or a derivative thereof and may improve the solubility of some donor acyl esters. In fact, the use of hydrophobic solvents is generally known and usually essential to improve the stability and activity of the enzyme to an acceptably high level, as described in for example J.O. Rich et al., Biotechnology and Bioengineering, 45 (1995) p. 426-434. It is therefore even more surprising that in the process according to the invention the presence of such hydrophobic solvents is not necessary. In fact the process according to.the invention results in superior selectivities and yields even when performed in non-nucleophilic, polar solvents such as pyridine or mixtures thereof.

The reaction is preferably carried out at a reaction temperature between 0 and 100 °C, the most suitable reaction temperature depending on the type of enzyme and the donor acyl ester used. At temperatures below 0 °C the reaction will be relatively slow, while at temperatures higher than 100 °C the enzyme stability is affected significantly, resulting in a lower regioselectivity and enzyme efficiency. More preferably the reaction is performed at a reaction temperature between 20 and 80 °C, most preferably at a reaction temperature between 30 and 70 °C.

The reaction time for achieving the desired high conversion level can vary within broad ranges and is dependent on for example the types, amounts and concentrations of the enzyme, sucrose or a derivative thereof and the donor acyl ester, the solvent(s) and the temperature. A skilled person can easily determine the most suitable reaction time for a particular combination of enzyme, sucrose or derivative thereof, donor acyl ester, solvent(s) and reaction conditions. Usually a reaction time of at least 1 h is aimed at, more preferably a reaction time of at least 6 h, and even more preferably of at least 12 h is aimed at. However, reaction times of more than one or two days, for instance, between 3 and 7 days, may still be suitable. In general reaction times will be shorter if the enzyme activity (in U/gram enzyme) is higher and/or if more enzyme is applied.

The reaction mixture may be kept in motion, for example, using a shaker or by stirring. If a shaker is used, the shaking speed is preferably between 10 and 1000 rpm, more preferably between 100 and 600 rpm, most preferably between 200 and 400 rpm. Shaking speeds higher than 1000 ppm may negatively affect the stability of the enzyme. Alternatively, the mixture can be passed over a column in which the solid and or immobilized enzyme catalyst is packed as a fixed bed.

If desired, the 6-O-acylsucroses obtained by the method according to the invention can be used as an intermediate product in a wide range of further reactions.

For example, the 6-O-acylsucroses can be further subjected to a chlorination reaction. Suitable methods for performing the chlorination reaction are disclosed in for example GB-B-2,079,749 (Vilsmeier reagents, sulphuryl chloride) and GB-A-2,195,632 (thionyl chloride/triphenylphosphine oxide).

In particular 6-O-acylsucroses can be used for the preparation of sucralose by subjecting 6-*O*-acylsucrose to subsequently a chlorination reaction using a Vilsmeier reagent, a peracylation reaction, for example in pyridine and a deesterification (hydrolysis) reaction, followed by isolation of sucralose, as described in for example WO-A-9960006. Sucralose may also be prepared using a method in which the peracylation reaction is omitted, as described in for example US-A- 4,980,463.

The invention will now be explained in more detail with reference to the following examples, without however being limited thereto in any way.

### Examples

### Starting materials

Information about the lipases used is listed in Table 1.

**Table 1. Information about the lipases used.**

| Species | Lipase | Supplier | Activity |
|---|---|---|---|
| *Pseudomonas cepacia* | Lipase P | Amano | ≥ 30.000 U/g powder |
| *Burkholderia gladioli* | Lipase PL | Meito Sangyo | 100.000U/g powder |
| | Lipase QL | Meito Sangyo | 30.000U/g powder |
| | Lipase QLM | Meito Sangyo | 60.000U/g powder |

Lipase P was assayed by the method disclosed in the product sheet provided by Amano; 1 mL of substrate solution (5% bovine albumine dissolved in 20 mL 0.1 M phosphate buffer pH=7 mixed with 3 mL of Fatgen (sesame oil emulsion)) mixed with 0.5 mL 0.1 M phosphate buffer pH=7 is incubated at 37 °C for 5 min in a closed test tube. 0.5 mL of diluted enzyme solution is added and the mixture is incubated at 37°C for 20 min. Subsequently 5 mL of stop solution (10 volumes of n-heptane, 40 volumes of isopropylalcohol and 1 volume of 2 N aq. sulphuric acid) is added and shaken, free fatty acids are extracted with 3 mL of n-heptane and 2 mL of distilled water. 3 mL of n-heptane layer is taken up and after adding 1 drop of Thymol Blue (1% in ethanol) as indicator, the solution is titrated with 0.01 N alcoholic KOH solution (0.8 g KOH in 10 mL distilled water diluted to 1 liter with 95% ethanol) in nitrogen gas currents (t mL). Controls are determined in the same way with distilled water (B mL). 1 unit (U) is defined as the lipase activity to free 1 µmole of fatty acid per 1 minute. Lipolytic activity (U/g)= 1.000 x 0.01 x 2 x 1/20 x 1.327 x (t - B) x D in which D = multiple of dilution.

The lipases from *Surkholderia gladioli* were assayed by the method of Kokusho et al. (Agric. Biol. Chem. 46 (1982), 1159-1164) using olive oil emulsified with polyvinyl alcohol as a substrate (Naks Y., Yukagaku 36 (1987), 821-825), 1 U corresponds to the amount of enzyme, which liberates 1 µmol fatty acid per min at 37 °C.

### Example I-III. Preparation of 6-O-acetylsucrose using isopropenyl acetate as the donor acyl ester and lipases produced by a micro-organism from a Burkholderia gladioli species

6-*O*-acetylsucrose was prepared using different lipases produced by a micro-organism from a *Burkholderia gladioli* species and isopropenyl acetate as the donor acyl ester. The lipases were lyophilized as described in Examples V-VI before use to remove water if present in the commercial samples. For Lipase PL and Lipase QL, to a mixture of sucrose (0.25 g, 0.73 mmol) and lipase (0.25 g) in pyridine (10 mL) isopropenylacetate (0.46 g, 4.6 mmol) was added (donor acyl ester : sucrose = 6 : 1 mol/mol). In the case of Lipase QLM higher concentrations (2x) of the reactants were applied: 0.50 g of sucrose, 0.50 g of lipase and 0.87 g of isopropenylacetate. The reaction vessel was closed and the reaction mixture was maintained at 37 °C and shaken at 300 rpm. Conversion to 6-O-acetylsucrose was followed in time by determining the concentration of 6-O-acetylsucrose via Thin Layer Chromatography (TLC), using silica gel, and quantitatively by High Performance Liquid Chromatochraphy (HPLC) on a Nucleosil^{®}-NH₂ HPLC column, with detection of the components on a refractometer. Eluent: acetonitrile / water (85/15 v/v). Before sampling, the shaking of the reaction vessel was stopped for 30 min, to allow the enzyme to deposit on the bottom of the reaction vessel so that clear samples could be taken. Samples of 50 µL were taken from the reaction mixture and diluted with 450 µL eluent before injection. Results are presented in Table 2.

**Table 2. Preparation of 6-O-acetylsucrose using isopropenyl acetate as the donor acyl ester and a lipase produced by a micro-organism from a Burkholderia gladioli species.**

| Example | Enzyme catalyst | Results | |
|---|---|---|---|
| | | Reaction time (h) | Conversion to 6-O-acetylsucrose (mol%) |
| I | Lipase PL | 4 | 1 |
| | (lyophilized) | 76 | 10 |
| | | 290 | 20 |
| II | Lipase QL | 4 | 11 |
| | (lyophilized) | 76 | 50 |
| | | 290 | 59 |
| III | Lipase QLM | 20 | 29 |
| | (lyophilized) | 48 | 48 |
| | | 92 | 59 |

Table 2 shows that three lipases produced by a micro-organism from a *Burkholderia gladioli* species, in particular lipase QL and lipase QLM, are surprisingly active in the conversion of sucrose to 6-*O*-acetylsucrose.

### Comparative Example A. Preparation of 6-O-acetvlsucrose using isopropenyl acetate as the donor acyl ester and Lipase P (Amano)

A reaction was performed using Lipase P (Amano) as the enzyme, applying the same reaction conditions as in Examples I-III. Lipase P (Amano) is produced by a micro-organism from a *Pseudomonas cepacia* species. The results are given in Table 3.

**Table 3. Preparation of 6-O-acetylsucrose using isopropenyl acetate as the donor acyl ester and Lipase P as the biocatalyst**

| Enzyme catalyst | Results | |
|---|---|---|
| | Reaction time (h) | Conversion to 6-O-acetylsucrose (mol%) |
| Lipase P | 2 | 0 |
| (lyophilized) | 24 | 1 |
| | 50 | 2 |
| | 72 | 2 |
| | 96 | 3 |

### Example IV (IV.1 - IV.3) and Comparative Examples B.1 - B.23. Preparation of 6-O-acetylsucrose using isopropenyl acetate as the donor acyl ester and lipases produced by a micro-organism from a Burkholderia gladioli species (resp. from other origin for the Comparative Examples) under conditions different from Examples I-III

6-O-acetylsucrose was prepared using lipases produced by a micro-organism from a *Burkholderia gladioli* species (Examples IV.1-IV.3), respectively lipases from a different origin (Comparative Examples B.1-8.23) and isopropenyl acetate as the donor acyl ester. For each of these Examples and Comparative Examples, to a mixture of sucrose (0.5 g, 1.46 mmol) and lipase (0.5 g) in pyridine (10 mL) was added isopropenylacetate (0.45 g, 4.5 mmol) (donor acyl ester : sucrose = 3 1 mol/mol). The reaction vessel was closed and the reaction mixture was maintained at 37 °C and shaken at 300 rpm for 70 hours. Conversion to 6-O-acetylsucrose and by-products, e.g. sucrose mono-, di- and triesters, fructose, glucose, glucose esters was determined qualitatively via Thin Layer Chromatography (TLC), using silica gel, and quantitatively by High Performance Liquid Chromatochraphy (HPLC) on a Nucleosil^{®}-NH₂ HPLC column, with detection of the components on a refractometer. Eluent: acetonitrile / water (85/15 v/v). After shaking for 70 hours, and before sampling, the shaking of the reaction vessel was stopped for 30 min, to allow the enzyme to deposit on the bottom of the reaction vessel so that clear samples could be taken. Samples of 50 µL were taken from the reaction mixture and diluted with 450 µL eluent before injection. Results are presented in Table 4.

**Table 4. Preparation of 6-O-acetylsucrose using isopropenyl acetate as the donor acyl ester and various lipases obtained from different origin.**

| Example, respectively Comparative Example | Origin of lipase | Biocatalyst | Supplier | Result (conversion to 6-O-acetylsucrose in %) other remarks |
|---|---|---|---|---|
| IV.1 | *Burkholderia gladioli* | Lipase PL | Meito Sangyo | 12 |
| IV.2 | *Burkholderia gladioli* | Lipase QL | Meito Sangyo | 18 |
| IV.3 | *Burkholderia gladioli* | Lipase QLM | Meito Sangyo | 37 |
| B.1 | *Candida lipolytica* | Lip F6 | Enzymatix | None |
| B.2 | *Candida lipolytica* | Lipase L10 | Amano | None |
| B.3 | *Candida lipolytica* | Lipase C. *lipolytica* | Biocatalysts | None |
| B.4 | *Humicola lanuginosa* | Lipase CE Amano10 | Amano | < 5, not 6-selective |
| B.5 | *Humicola lanuginosa* | Lipozyme TL IM | Novo | None |
| B.6 | *Humicola lanuginosa* | Chirazyme L-8 | Roche | <5 |
| B.7 | *Humicola lanuginosa* | Lip F13 | Enzymatix | <5 |
| B.8 | *Humicola lanuginosa* | Lipase H. *lanuginosa* | Biocatalysts | Not 6-selective; invertase activity |
| B.9 | *Humicola lanuginosa* | SP 523 | Novo | None |
| B.10 | *Mucor javanicus* | Lipase M Amano10 | Amano | None |
| B.11 | *Mucor miehei* | Lipase RM IM (= IM20) | Novo | None |
| B.12 | *Mucor miehei* | SP 225 | Novo | None |
| B.14 | *Mucor miehei* | Lip F7 | Enzymatix | None |
| B.15 | *Mucor miehei* | Lipase *Mucor miehei* | Biocatalysts | <5 |
| B.16 | *Mucor miehei* | SP 254 | Novo | None |
| B.17 | *Pseudomonas cepacia* | Lipase AK Amano | Amano | Not 6-selective and invertase activity |
| B.18 | *Pseudomonas cepacia* | Lipase PS D Amano I | Amano | 3 and 1% of 1'-O-acetylsucrose |
| B.19 | Porcine pancreas | Lipase PPL | Fluka | None |
| B.20 | Porcine pancreas | Lipase PPL | USBC | None |
| B.21 | *Rhizopus oryzae* | Lip F3 | Enzymatix | None |
| B.22 | *Rhizopus oryzae* | Lipase D Amano20 | Amano | < 5 |
| B.23 | *Rhizopus oryzae* | Lipase N Amano cone. | Amano | None |

Table 2 shows that lipases produced by a micro-organism from a *Burkholderia gladioli* species, are much more active and selective in the conversion of sucrose to 6-O-acetylsucrose than lipases from other origin.

### Examples V-VI. influence of lyophilization of the enzyme on the enzyme activity and selectivity

The influence of lyophilization of the lipase on its catalytic activity was determined for Lipase QLM using isopropenyl acetate as the donor acyl ester. Herein lyophilization was carried out by storing the lipase (as a powder) together with sucrose in a freezer at -80 °C for 30 min. Subsequently the lipase was dried in a freeze dryer overnight. Pyridine (10 mL) and isopropenylacetate (0.46 g, 4.6 mmol) were added (donor acyl ester: sucrose = 3 : 1 mol/mol) to the lyophilized mixture of sucrose (0.50 g, 1.46 mmol) and lipase (0.50 g). Further reaction conditions were the same as those in Example I. The results for Lipase QLM as received and after lyophilization are given in Table 5.

**Table 5. Influence of lyophilization of the enzyme on the acylation of sucrose to 6-O-acetylsucrose.**

| Example | Enzyme catalyst | Results | |
|---|---|---|---|
| | | Reaction time (h) | Conversion to 6-O-acetylsucrose (%) |
| V | Lipase QLM | 24 | 14 |
| | as received | 48 | 21 |
| | | 174 | 37 |
| VI | Lipase QLM | 24 | 25 |
| | lyophilized | 48 | 34 |
| | | 174 | 48 |

The results in Table 5 show that lyophilization of Lipase QLM in the presence of sucrose as the lyoprotectant has a positive effect on its catalytic activity in the acylation of sucrose to 6-*O*-acylsucrose.

### Example VII-IX. Acylation of sucrose with different donor acyl esters in the presence of Lipase QLM.

Different donor acyl esters were tested for the acylation of sucrose in the presence of Lipase QLM as the enzyme catalyst. Lipase QLM was used as received. To a mixture of sucrose (0.50 g, 1.46 mmol) and lipase (0.50 g) in pyridine (10 mL) donor acyl ester (8.7 mmol) was added (donor acyl ester: sucrose = 6 : 1 mol/mol). Further reaction conditions were the same as those in Examples I-III. The results are listed in Table 6.

**Table 6. Acylation of sucrose to 6-O-acylsucrose with different donor acyl esters in the presence of Lipase QLM.**

| Example | Donor acyl ester | Result | |
|---|---|---|---|
| | | Reaction time (h) | Conversion to 6-O-acetylsucrose (%) |
| VII | Isopropenyl acetate | 20 | 29 |
| | | 48 | 48 |
| VIII | Vinyl propionate | 20 | 46 |
| | | 48 | 58 |
| IX | Vinyl butyrate | 20 | 48 |
| | | 48 | 65 |

### Example X-XI. Influence of the molar ratio donor acyl ester: sucrose on the vield

To investigate the influence of the molar ratio between the donor acyl ester and sucrose on the yield, experiments were performed using lipase QLM as received, isopropenyl acetate as the donor acyl ester and donor acyl ester: sucrose molar ratios of 3:1 and 6:1, respectively, at a temperature of 37°C, using concentrations of 146 mM (sucrose) and 50g/L (enzyme). The results are given in Table 7.

**Table 7. Influence of the molar ratio donor acyl ester: sucrose on the yield.**

| Example X | | Example XI | |
|---|---|---|---|
| Donor acyl ester : sucrose 3:1 | | Donor acyl ester : sucrose 6:1 | |
| Reaction time (h) | Conversion to 6-*O*-acetylsucrose (mol%) | Reaction time (h) | Conversion to 6-O-acetylsucrose (mol%) |
| 24 | 14 | 20 | 29 |
| 48 | 21 | 48 | 48 |
| 174 | 37 | 92 | 59 |

Table 7 shows that when isopropenyl acetate and Lipase QLM are used, an increase of the donor acyl ester : sucrose ratio from 3:1 to 6:1 results in a 2-fold increase of reaction speed.

### Example XII-XIII. Influence of the reaction temperature

The influence of the reaction temperature on the enzymatic acylation is illustrated by performing tests with Lipase QLM as the enzyme catalyst and isopropenyl acetate as the donor acyl ester (donor acyl ester: sucrose = 3 : 1) at 37 and 60 °C, respectively, using concentrations of 146 mM (sucrose) and 50g/L (enzyme). No significant difference was observed between the reaction rates at 37 and 60 °C.

### Example XIV. Preparation, isolation and molecular characterization of 6-O-acylsucrose via enzymatic acylation of sucrose.

To a mixture of sucrose (1.0 g, 0.003 mol) and enzyme catalyst (1 g, lipase QL, Meito Sangyo, lot no. N3104) in pyridine (40 mL) was added isopropenyl acetate (2 mL, 0.02 mol, 6 molar equivalents). The reaction vessel was closed and the reaction mixture was maintained at 37 °C and shaken at 300 rpm for 18 days. A filtrate sample of the crude reaction mixture was analyzed by HPLC and was found to contain 6-O-acetylsucrose as the main product and minor amounts of byproducts such as 1'-O-acetylsucrose, 6'-O-acetylsucrose and sucrose-di- and tri-acetates. The enzyme was separated from the mixture using a G-3 glass funnel and washed with toluene. The filtrate and the toluene washing were evaporated separately (rotary evaporator, 40°C) to remove the solvents. The resulting oils were cooled to -80 °C and freeze dried using an oil pump. Both fractions were analyzed using TLC on silica gel (eluent: EtOAc/MeOH / H₂O=75/25/5 v/v/v) and were found to contain mainly sucrose-monoacetate (Rₜ = 0.3). Pyridine was removed from the combined oil fractions by co-evaporation with toluene (2 x 30 mL) *in vacuo* at 40 °C. The remaining volatiles were evaporated with a vacuum pump. The brown solid residue was further purified using column chromatography on silica gel (eluent: CHCl₃ / methanol / acetic acid water=60/30/3/5 v/v/v/v), Tetrahedron, vol. 51, no 13, p 3881, 1995).

6-*O*-acetylsucrose (0.17 g, 0.4 mmol) was obtained in high purity (no by-products could be observed in the ¹H NMR and ¹³C NMR spectra). The product was characterized by ¹H and ¹³C NMR spectroscopy recorded at 300 and 75 MHz, respectively. ¹H NMR (D₂O) (δ in ppm): 2.05 (s, 3H), 3.36 (t, J = 9.6 Hz, 1H), 3.48 (dd, J = 9.9, 3.8 Hz, 1H), 3.57 (s, 1 H), 3.67 (t, J = 9.6 Hz, 1H), 3.70 - 3.74 (m, 2H), 3.76 - 3.84 (m, 1H), 3.94 (t, J = 8.4 Hz, 1 H), 3.97 - 4.01 (m, 1H), 4.13 (d, J = 8.7 Hz, 1H), 4.19 (dd, J = 12.3, 5.1 Hz, 1H), 4.30 (dd, J = 12.3, 2.3 Hz, 1H), 5.32 (d, J = 3.8 Hz, 1H). ¹³C NMR (D₂O) 20.58 (q), 61.72 (t), 62.99 (t), 63.94 (t), 69.92 (d), 70.60 (d), 71.28 (d), 72.69 (d), 74.52 (d), 76.66 (d), 81.77 (d), 92.32 (d), 104.06 (d), 174.45 (d).

## Claims

1. Process for the enzymatic preparation of 6-*O*-acylsucrose (Figure 1 with R representing an optionally substituted (hetero)alkyl or (hetero)aryl group having from 1 to 50 carbon atoms) or a derivative thereof 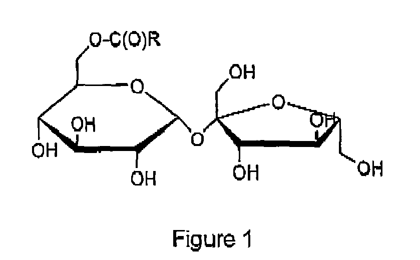 by selective monoacylation of sucrose on the 6-position with a donor acyl ester of formula R¹R²C=CR³-O-C(O)R, in which R¹, R², and R³ each independently represent hydrogen, an optionally substituted (hetero)alkyl group or an optionally substituted (hetero)aryl group with for example 1-50 C atoms and in which R has the meaning defined above, or of formula R⁴-O-C(O)R, in which R⁴ is a halogenated alkyl group of 2-50 carbon atoms in which at least the 2-position of the alkyl group is substituted with one, two or three halogens, in the presence of an enzyme and at least one non-nucleophilic organic solvent, **characterized in that** the enzyme is lipase enzyme selected from the group of
(i) lipases obtainable from a micro-organism from a *Burkholderia gladioli* species; or
(ii) any such *Burkholderia gladioli* lipase enzyme produced and over-expressed in another suitable micro-organism; or
(iii) mutants of the lipases from (i) and/or (ii) having selective acylation activity to form 6-*O*-acylsucrose in acylation of sucrose.

2. Process according to claim 1, **characterized in that** the lipase is chosen from the group consisting of Lipase PL, Lipase QL and Lipase QLM.

3. Process according to claim 1 or claim 2, **characterized in that** the donor acyl ester is chosen from the group consisting of isopropenyl acetate, vinyl propionate and vinyl butyrate.

4. Process according to any one of claims 1-3, **characterized in that** the ratio between the donor acyl ester and sucrose or a derivative thereof is in the range between 2:1 and 10:1.

5. Process according to any one of claims 1-4, **characterized in that** at least one non-nucleophilic organic solvent is chosen from the group consisting of amines, amides, sterically hindered alcohols, and sulfoxides, or mixtures thereof.

6. Process according to claim 5, **characterized in that** at least one non-nucleophilic organic solvent is pyridine.

7. Process according to any one of claims 1-6, **characterized in that** the process is performed at a temperature between 30 and 70 °C.

8. Process according to any one of claims 1-7, **characterized in that** the 6-*O-*acylsucrose obtained is further subjected to a chlorination reaction.

9. Process according to claim 8, **characterized in that** the chlorination is carried out using a Vilsmeier reagent.

10. Process according to claim 8 or claim 9, **characterized in that** the chlorination reaction is followed by a deesterification (hydrolysis) reaction, followed by isolation of sucralose.

11. Process according to claim 8 or claim 9, **characterized in that** the chlorination reaction is followed by a peracylation and a deesterification (hydrolysis) reaction, followed by isolation of sucralose.
